# EUROPEAN PATENT APPLICATION

(11) **EP 2 022 527 A1**
(43) Date of publication of application: **11.02.2009**
(21) Application number: 07113966.1
(22) Date of filing: 07.08.2007
(51) Int. Cl.: A61M 15/00

(54) **Inhalation device for drug inhalation therapy**

(71) Applicant: L'AIR LIQUIDE, Société Anonyme pour l'Etude et l'Exploitation des Procédés Georges Claude, 75007 Paris (FR)
(72) Inventor: Birngruber, Reginald, 23564, Lübeck (DE); Texereau, Joelle, 75013, Paris (FR); Apiou, Gabriela, 75014, Paris (FR); Haussermann, Sabine, 78000, Versailles (FR)
(74) Representative: Pittis, Olivier

(57) **Abstract**

Inhalation device for drug inhalation therapy comprising a main body (2) defining a chamber for receiving at least a gas and a drug to be inhaled by a user, said main body (2) having an outlet (13) for delivering the gas and drug mixture contained in the chamber (12) to the user's airways, characterized in that it further comprises cartridge-connection means (1) for connecting at least a cartridge (6) containing a gas under pressure to the main body (2), and releasing means (8) for allowing the passage of the under-pressure gas from the cartridge (6) to the chamber (12) when a cartridge (6) is connected to the cartridge-connection means (1).

## Description

The invention concerns an inhalation device connected to a gas cartridge useable for inhalation therapies.

Inhalation devices, also called "spacers", are portable devices that do not need any power supply for working, and are currently used to improve the deposition of drugs delivered by metered dose inhalers (MDI) in the lung of patients.

Inhalation devices or spacers are widely used by patients who have difficulties to use MDI sprays alone, like children or elderly patients who can not coordinate inspiration effort with the triggering of the device. Also, they are used for drugs, like inhaled corticosteroids which have unfavourable side effects if deposited in the oropharyngeal region.

A spacer comprises a main chamber defining a chamber having, at one extremity, an opening in which a MDI is inserted for delivering the drug stored in the MDI into the chamber of the spacer which contains air as a carrier gas and, at the other extremity, an outlet with a mouthpiece that allows the patient to inhale the air/drug mixture formed in the chamber of the spacer. In such known spacers, the carrier gas is always air.

However, one problem is that with such known spacers, the inhalation by the patient can be difficult, especially for patients having a low breathing ability, like children or patients with severely impaired lung function for instance, and further the deposition of the drug in the patient's airways and/or lung is not always very efficient and sometimes rather bad, especially with the above mentioned kinds of patients because their inspiratory volume is low and/or their airways are obstructed. Therefore, inserting a different carrier gas than air for the drug particles could be favourable for carrying the particles deeper into the lungs, and enable drug targeting of the lung periphery.

Furthermore, the existing spacers are not always very practical to use.

The goal of the present invention is to propose an improved spacer that leads to an easier and faster inhalation and an improved deposition of drug in the small airways, in healthy and compromised lung morphologies.

A solution according to the present invention is an inhalation device for drug inhalation therapy comprising a main body defining a chamber for receiving at least a gas and a drug to be inhaled by a user, said main body having an outlet for delivering the gas and drug mixture contained in the chamber to the user's airways, characterized in that it further comprises :
- cartridge-connection means for connecting at least a cartridge containing a gas under pressure to the main body, and
- releasing means for allowing the passage of the under-pressure gas from the cartridge to the chamber when a cartridge is connected to the cartridge-connection means.

Besides, the device of the invention can comprise one or several of the following additional features:
- said releasing means are adapted to put the interior of the cartridge in fluid communication with the chamber thereby releasing gas from the cartridge to the spacer's chamber when the cartridge is connected to said cartridge-connection means.
- said releasing means comprises an hollow needle, in particular a sharp or a tube-form needle.
- a gas-cartridge is connected to the connection means, in particular a disposable or refillable cartridge.
- the cartridge contains a gas chosen among He, O₂ or a mixture thereof.
- the cartridge further contains an inhalable drug in liquid or powder form.
- the cartridge comprises at least a first and second compartments, the first compartment containing the gas or gas mixture, the second compartment containing the drug.
- it comprises at least two cartridges, one of said cartridges containing the gas or gas mixture, and the other containing the drug.
- the cartridges or compartments are arranged in such a way that, when they are connected to the connection means, the gas delivered by the first cartridge or first compartment to the chamber draws at least some of the liquid or powder drug contained in the second cartridge or compartment thereby obtaining a nebulization of said liquid drug or a dispersion of said dry-powder drug.
- the main body comprises an additional opening for insertion therein of a metered dose inhaler.
- it further comprises fixing means for maintaining the cartridge in its position in the connection means, in particular a bracket or similar.
- the connection means comprise threatening means for threating/screwing the cartridge to the main body.
- it further comprises sealing means arranged between the cartridge and the main body of the device.
- the main body comprises a recess portion adapted to at least partially receive at least a cartridge, preferably said recess portion is formed in the wall of main body that projects into the chamber.
- the connection means and/or the releasing means are arranged in said recess portion.
- the outlet comprises a mouthpiece.

The present invention will be described now in details in references to the enclosed figures among them :
- Figure 1 shows a schematic view of an inhalation device or spacer according to the present invention,
- Figure 2 represents a enlarged view of a gas cartridge inserted in the spacer of Figure 1 according to a first embodiment,
- Figure 3 represents a second embodiment of a gas cartridge inserted in the spacer of Figure 1.

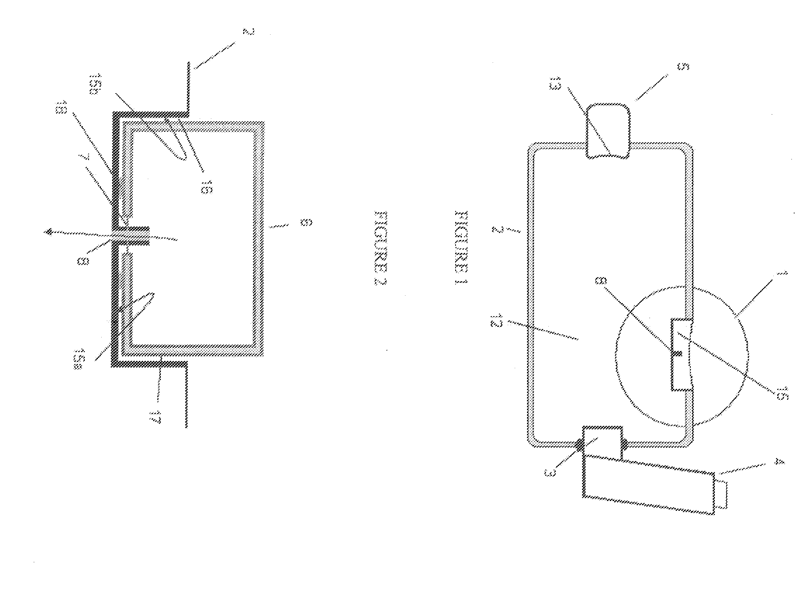

Figure 1 represents a schematic view of a spacer useable in various drug inhalation therapies according to the present invention comprising a main body 2 defining a chamber 12 having an internal volume for receiving a gas or gas mixture and further a drug to be inhaled by a patient.

The main body 2 can have an elongated shape and be made of polymer, metal or any other material. Commonly, spacer's main body 2 are made in plastic, sometimes coated with carbon, for the benefit of the electrostatic properties.

Further, the internal volume of the chamber 12 is typically less than 1 litre, preferably less than 0,5 litre.

Besides, the main body 2 of the spacer 12 has an outlet 13 connected to a mouthpiece 5 for delivering the gas and drug mixture formed in the chamber 12 to the airways of the patient.

Cartridge-connection means 1 for connecting the gas cartridge 6 to the main body 2 of the spacer are arranged on the main body 12 of the spacer. These cartridge-connection means 1 can be any suitable mechanism adapted for receiving a gas cartridge 6 and maintaining it in a fixed position, i.e. connected to the spacer.

Releasing means 8 are also arranged on the spacer body for allowing the passage of the gas under-pressure from the cartridge 6 to the chamber 12 when the cartridge 6 is connected to the cartridge-connection means 1.

The realising means 8 may include an hollow needle, sharp or not (lube), that is able to penetrate into the cartridge 6 thereby putting the internal volume of the cartridge 6 comprising the gas in fluid communication with the chamber 12 so that the gas is released from the cartridge to the spacer's chamber 12 when the cartridge 6 is connected to said cartridge-connection means 1. The needle 8 is designed to be able to pierce the wall itself of the cartridge 6 or a specific area of said wall, i.e. an area or part made of a weaker and/or easy-to-pierce material that can be pierced by the needle 8 or similar when said needle 8 is forced into it. For instance, said weaker and/or easy-to-pierce material can be a membrane 7 that is arranged to normally close an opening made in the cartridge 6 and which can be perforated by the needle 8 or similar for liberating the gas in the chamber 12.

As shown, the main body 2 comprises a recess portion 15 adapted to at least partially receive at the cartridge 6, i.e. the cartridge 6 is partially inserted into said recess 15 as shown on Figure 2. The realising means 8 are arranged on the bottom 15a of said recess 15.

Preferably, said recess portion 15 is arranged in the peripheral wall of main body which projects into the chamber 12 thereby forming said recess 15 and it is configured to be adapted to the size and dimensions of one or several types of cartridges.

Further, the cartridge-connection means 1 can comprise a fine thread portion 16 arranged on the peripheral internal surface 15b of the recess 15, whereas a complementary fine thread portion 17 is arranged on the external peripheral wall of the cartridge 6 in order to screw the cartridge 6 into the fine thread portion 16 of the recess 15.

Optionally, sealing means 18 can be arranged between the cartridge 6 and the main body 2 of the spacer, preferably in the recess 15, in order to limit or avoid any gas leakage as shown on Figure 2.

Moreover, as shown on Figure 1, the main body 2 of the spacer can include an additional opening 3 for insertion therein of a classical MDI 4, i.e. metered dose inhaler. Indeed, the high pressure gas in the capsule can drive an MDI attached or implanted to the spacer and aerosolize a drug simultaneously to the release of the gas as further explained below. However, the spacer is not intended to be used only with MDI, but could also be used with dry powder inhalers.

Figure 2 shows a first embodiment of the invention of a gas cartridge 6 inserted in the recess 15 formed by a projection of the peripheral wall of the chamber 12 toward the interior of the chamber 12. In this embodiment, the cartridge 6 contains a gas or gas mixture such as helium or a helium/oxygen mixture and eventually a drug in powder or liquid form that can be dispersed or atomized in the chamber 12 once the gas is released in said chamber 12.

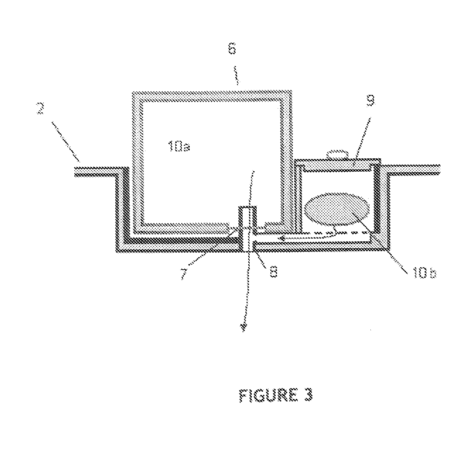

Figure 3 represents a second embodiment of a spacer according to the present invention, wherein 2 cartridges 6, 9 are connected to the main body 2 of the spacer, one of said cartridges 6 containing the gas or gas mixture, whereas the other cartridge or compartment 9 containing the drug.

In other words, both cartridges 6, 9 are arranged in such a way that, when they are connected to the connection means 8, the gas delivered by the first cartridge 6 to the chamber 12 draws at least some of the liquid or powder drug contained in the second cartridge 9 or compartment 10b thereby obtaining a nebulization of said liquid drug or a dispersion of said dry-powder drug.

Indeed, in that embodiment, the circulation of the gas that goes out of the gas cartridge 6 sucks up the liquid or powder contained in the second compartment 10b.

Whatever the embodiment is, it is advocated to use a gas cartridge containing a helium or a helium/oxygen mixture and introduce it in the chamber 12 of the spacer in lieu of air as the drug-carrier gas as He or He/O₂ mixtures exhibit better flow dynamical properties and allow to a better and more efficient drug deposition in the deep lung and in obstructed airways.

Technically, a He/O₂ mixture is provided to the chamber 12 by a small and highly compressed gas capsule 6 which is connectable to the internal volume or chamber 12 of the spacer, in which the compressed gas mixture is released. The gas capsule or cartridge 6 can be opened to the spacer volume 12 reversibly or irreversibly. In the latter case, the gas capsule 6 is disposable.

Further, it can contain pure compressed He or He/O₂ or a combination of compressed He or HeO₂ with one or several liquid or dry-powder drug substances.

The spacers or inhalation device of the invention can be used for inhaling for instance corticosteroids in paediatric asthma, which has to be deposited deeper in the lungs ; antiinflammatory drugs, bronchodilators, anti-tumoral agents, anti-viral drugs or antibiotics for respiratory diseases ; and systemic drugs delivered by the inhaled route, like insulin.

## Claims

1. Inhalation device for drug inhalation therapy comprising a main body (2) defining a chamber for receiving at least a gas and a drug to be inhaled by a user, said main body (2) having an outlet (13) for delivering the gas and drug mixture contained in the chamber (12) to the user's airways, **characterized in that** it further comprises :
- cartridge-connection means (1) for connecting at least a cartridge (6) containing a gas under pressure to the main body (2), and
- releasing means (8) for allowing the passage of the under-pressure gas from the cartridge (6) to the chamber (12) when a cartridge (6) is connected to the cartridge-connection means (1).

2. Device according to claim 1, **characterized in that** said releasing means (8) are adapted to put the interior of the cartridge (6) in fluid communication with the chamber (12) thereby releasing gas from the cartridge to the spacer's chamber when the cartridge (6) is connected to said cartridge-connection means (1).

3. Device according to any one of the preceding claims, **characterized in that** said releasing means (8) comprises an hollow needle (8), in particular a sharp or a tube-form needle.

4. Device according to any one of the preceding claims, **characterized in that** a gas-cartridge (6) is connected to the connection means (8), in particular a disposable or refillable cartridge.

5. Device according to any one of the preceding claims, **characterized in that** the cartridge (6) contains a gas chosen among He, O₂ or a mixture thereof.

6. Device according to any one of the preceding claims, **characterized in that** the cartridge (6) further contains an inhalable drug in liquid or powder form.

7. Device according to any one of the preceding claims, **characterized in that** the cartridge (6) comprises at least a first and second compartments (10a, 10b), the first compartment (10a) containing the gas or gas mixture, the second compartment (10b) containing the drug.

8. Device according to any one of the preceding claims, **characterized in that** it comprises at least 2 cartridges (6, 9), one of said cartridges (6) containing the gas or gas mixture, and the other (9) containing the drug.

9. Device according to any one of the preceding claims, **characterized in that** the cartridges (6, 9) or compartments (10a, 10b) are arranged in such a way that, when they are connected to the connection means (8), the gas delivered by the first cartridge (6) or first compartment (10a) to the chamber (12) draws at least some of the liquid or powder drug contained in the second cartridge (9) or compartment (10b) thereby obtaining a nebulization of said liquid drug or a dispersion of said dry-powder drug.

10. Device according to any one of the preceding claims, **characterized in that** the main body comprises an additional opening (3) for insertion therein of a metered dose inhaler (4).

11. Device according to any one of the preceding claims, **characterized in that** it further comprises fixing means (17) for maintaining the cartridge in its position in the connection means, in particular a bracket or similar.

12. Device according to any one of the preceding claims, **characterized in that** the connection means comprise threatening means (16) for threating/screwing the cartridge (6) to the main body (2).

13. Device according to any one of the preceding claims, **characterized in that** it further comprises sealing means (18) arranged between the cartridge (6) and the main body (2) of the device.

14. Device according to any one of the preceding claims, **characterized in that** the main body (2) comprises a recess portion (15) adapted to at least partially receive at least a cartridge (6), preferably said recess portion (15) is formed in the wall of main body (2) that projects into the chamber (12).

15. Device according to any one of the preceding claims, **characterized in that** the connection means and/or the releasing means (8) are arranged in said recess portion (15).

16. Device according to any one of the preceding claims, **characterized in that** the outlet (13) comprises a mouthpiece (5).
